# EUROPÄISCHE PATENTANMELDUNG

(11) **EP 0 668 035 A1**
(43) Veröffentlichungstag der Anmeldung: **23.08.1995**
(21) Anmeldenummer: 95101582.5
(22) Anmeldetag: 07.02.1995
(51) Int. Cl.: A45D 34/04, A61M 35/00

(54) **Vorrichtung zum Auftragen und Verteilen von Salben, Lotionen, Flüssigkeiten o.dgl. auf Körperpartien**

(30) Priorität: 21.02.1994 DE 9402781 U
(71) Anmelder: Marx, Roland, 65199 Wiesbaden (DE)
(72) Erfinder: Marx, Birgit, 42549 Velbert (DE)

(57) **Zusammenfassung**

Es handelt sich um eine Vorrichtung zum Auftragen und Verteilen von Salben, Lotionen, Flüssigkeiten o.dgl. auf Körperpartien, mit einem Tragbügel, an dessen einem Ende ein Handgriff vorgesehen ist und an dessen anderem freien Ende eine Rolle aufsetzbar und freidrehbar gelagert ist. Die Rolle trägt dabei an ihrer Umfangsfläche eine Beschichtung. Um die gattungsgemäße Vorrichtung so zu verbessern, daß diesselbe schwer zugänglichen Körperpartien angepaßt ist und dadurch die Handhabung der Vorrichtung wesentlich vereinfacht wird, weist der Tragbügel (2) mit seinem rechtwinklig zur Längsachse (9-9) der Rolle (4) angeordneten und den Handgriff (3) aufweisenden Bügelteil (10) eine der Bewegungsrichtung (11) der Rolle (4) angepaßte Biegung (12) auf.

## Beschreibung

Die Erfindung bezieht sich auf eine Vorrichtung gemäß der im Oberbegriff des Patentanspruchs 1 angegebenen Gattung.

Bei einer solchen aus der DE 93 14 138 U1 bekannten Vorrichtung ist der Handgriff lösbar mit dem Tragbügel verbunden, und zwischen Handgriff und Tragbügel ist ein Verlängerungsstab einsetzbar.

Der Erfindung liegt die Aufgabe zugrunde, die gattungsgemäße Vorrichtung so zu verbessern, daß dieselbe schwer zugänglichen Körperpartien angepaßt ist und dadurch die Handhabung der Vorrichtung wesentlich vereinfacht wird.

Diese Aufgabe wird bei einer gattungsgemäßen Vorrichtung erfindungsgemäß durch die Kennzeichnungsmerkmale des Patentanspruchs 1 gelöst.

Zweckmäßige Weiterbildungen der Erfindung sind in den Unteransprüchen gekennzeichnet.

Durch den ergonomisch geformten Tragbügel ist die Eigenhandhabung der Vorrichtung beim Auftragen von Salben, Lotionen, Flüssigkeiten, wie z.B. Sonnenöl und/oder -milch, Franzbranntwein, medizinische Salben o.dgl. auf schwer zugängliche Körperpartien, wie beispielsweise auf den Rücken, wesentlich vereinfacht. Die Abnehmbarkeit der Beschichtung bzw. auch der gesamten Rolle ermöglicht die Abstimmung der Beschichtung auf die sich durch unterschiedliche Viskositäten auszeichnenden, aufzutragenden Mittel, die einfach direkt auf die Beschichtung aufgetragen werden.

Die Schutzhülle trägt dafür Sorge, daß die Rolle bzw. Beschichtung in der Ruhepause zwischen zwei Einreibungen bzw. Auftragungen nicht eintrocknet oder verschmutzt. Bei längerem Lagern wäre ein Auswaschen der Rolle bzw. der Beschichtung von Vorteil, wonach die Schutzhülle anschließend als Schutz gegen Verschmutzungen auf der Rolle angebracht wird.

Ausführungsbeispiele der erfindungsgemäßen Vorrichtung sind in der Zeichnung dargestellt. Dabei zeigt
Fig. 1 eine Vorderansicht eines ersten Ausführungsbeispiels der Vorrichtung mit Metallbügel, bei der der Handgriff einen runden Querschnitt aufweist,
Fig. 2 eine Ansicht in Richtung des Pfeiles 11 in Fig. 1,
Fig. 3 einen Schnitt nach der Linie 111-111 in Fig. 1,
Fig. 4 eine Vorderansicht eines zweiten Ausführungsbeispiels der Vorrichtung ohne Metallbügel aber mit Verlängerungsstab, bei der der Handgriff einen ovalen Querschnitt aufweist,
Fig. 5 eine Ansicht in Richtung des Pfeiles V in Fig. 4,
Fig. 6 einen Schnitt nach der Linie VI-VI in Fig. 4,
Fig. 7 perspektivisch ein erstes Ausführungsbeispiel einer geöffneten Schutzhülle und
Fig. 8 perspektivisch ein zweites Ausführungsbeispiel einer geschlossenen Schutzhülle.

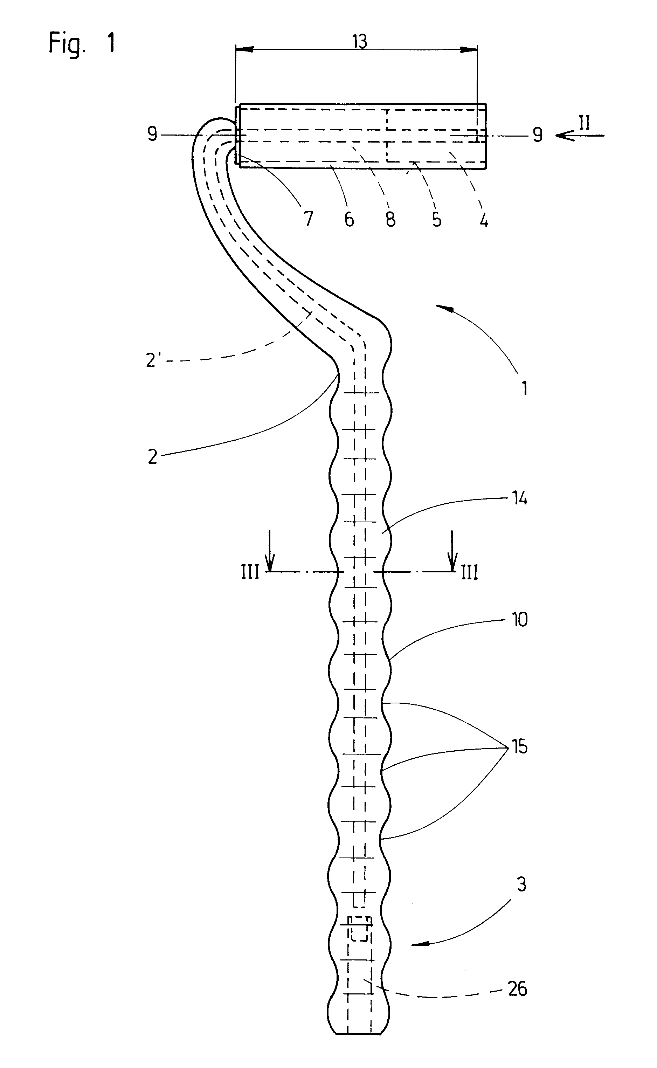

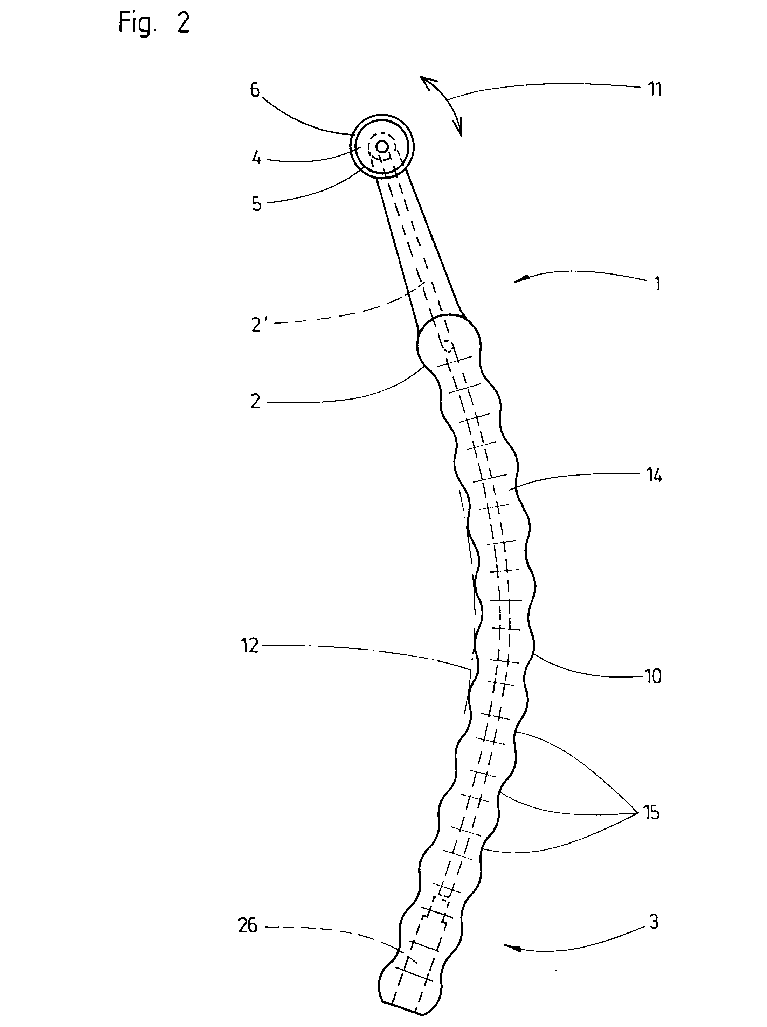

Die in den Fign. 1 bis 6 dargestellte Vorrichtung 1 zum Auftragen und Verteilen von Salben, Lotionen, Flüssigkeiten o.dgl. auf Körperpartien, weist einen Tragbügel 2 auf, an dessen einem Ende ein Handgriff 3 vorgesehen ist und an dessem anderen freien Ende eine Rolle 4 aufsetzbar und freidrehbar gelagert ist, die an ihrer Umfangsfläche 5 eine Beschichtung 6 trägt.

Die Beschichtung 6 ist von der Rolle 4 abnehmbar und somit auswechselbar und wird auf derselben durch Reibschluß gehalten. Dadurch ist es möglich, die Beschichtung 6, die entweder aus physiologisch und toxikologisch unbedenklichem Schaumstoff, Vlies oder Flock besteht, den Viskositäten und sonstigen Eigenschaften des jeweils aufzutragenden Mittels anzupassen. In einer Verkaufspackung kann die Vorrichtung vorzugsweise mit drei Beschichtungen 6, d.h. einmal Schaumstoff, einmal Vlies und einmal Flock angeboten werden, wobei auch Verkaufspackungen mit verschiedenartigen Schaumstoff-Beschichtungen oder Vlies-Beschichtungen oder auch Flock-Beschichtungen möglich sind.

Beim Aufsetzen der Beschichtung 6 wird diese einfach auf die Rolle 4 bis gegen eine Anschlagkante 7 geschoben, die an dem dem Tragbügel 2 zugewandten Ende der Rolle 4 oder an der Beschichtung 6 vorgesehen ist.

Auch ist es möglich, die Rolle 4 z.B. bei Beschädigungen oder geänderten Anforderungen insgesamt auszutauschen. Dazu wird die Rolle 4 einfach vom Tragbügel 2 abgezogen und eine neue Rolle 4 auf denselben aufgeschoben. Damit die Rolle 4 in ihrer Lage verbleibt, wird dieselbe durch Kraftschluß auf dem Tragbügel 2 gehalten, wobei die Drehbewegung der Rolle 4 durch eine in derselben integrierten Drehlagerung 8 gewährleistet ist.

Um die Handhabung der Vorrichtung 1 beim Auftragen von Salben, Lotionen, Flüssigkeiten o.dgl. wesentlich zu vereinfachen, weist der Tragbügel 2 mit seinem rechtwinklig zur Längsachse 9-9 der Rolle 4 angeordneten und den Handgriff 3 aufweisenden Bügelteil 10 eine der Bewegungsrichtung 11 der Rolle 4 angepaßte Biegung 12 auf, die einen Radius von etwa 150 mm hat.

Der Tragbügel 2 besteht entweder ganz aus Kunststoff (Fign. 4 bis 6) oder ist ein Metallbügel 2', der außerhalb des Aufsteckbereiches 13 der Rolle 4 eine Kunststoff-Ummantelung 14 aufweist (Fign. 1 bis 3). Dabei bildet das Ende der Kunststoff-Ummantelung 14 in Richtung des Aufsteckbereiches 13 der Rolle 4 eine Anschlagkante.

Der Tragbügel 2 bzw. die Kunststoff-Ummantelung 14 weist an dem rechtwinklig zur Längsachse 9-9 der Rolle 4 angeordneten Bügelteil 10 Griffmulden 15 auf.

Der Handgriff 3 weist, wie in den Fign. 1 bis 3 beispielsweise dargestellt, einen runden Querschnitt oder, wie in den Fign. 4 bis 6 beispielsweise dargestellt, einen ovalen Querschnitt auf. Der ovale Querschnitt des Handgriffes 3 hat den Vorteil, daß der Anwender der Vorrichtung 1 diesselbe ohne großen Kraftaufwand besser greifen kann.

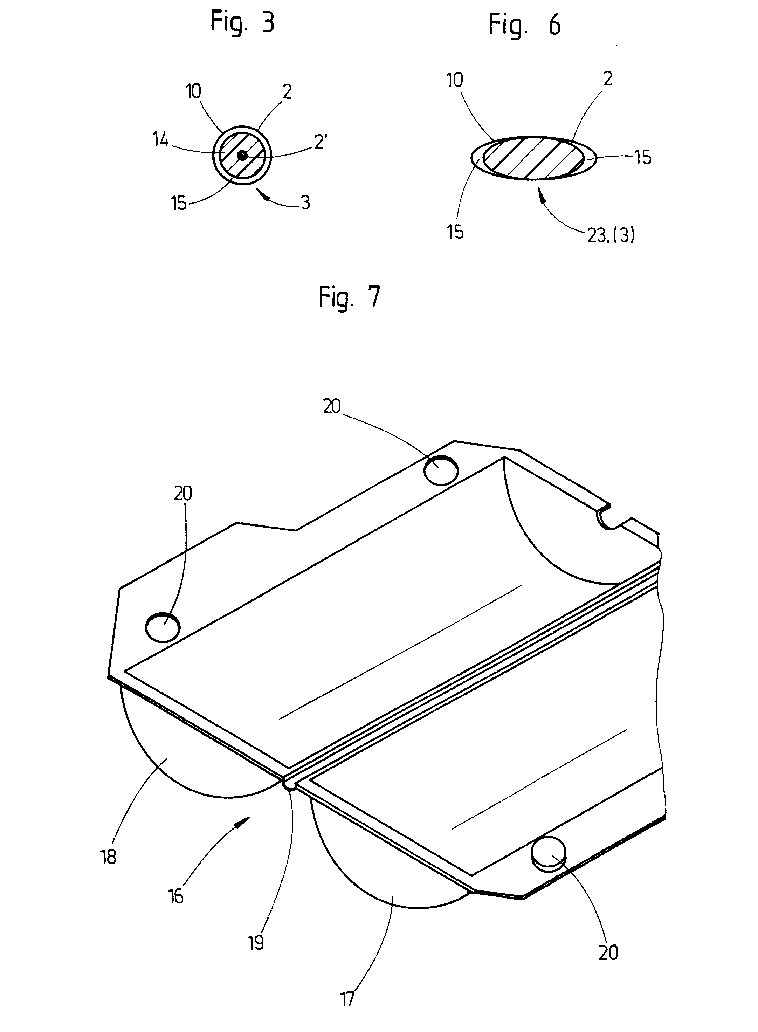

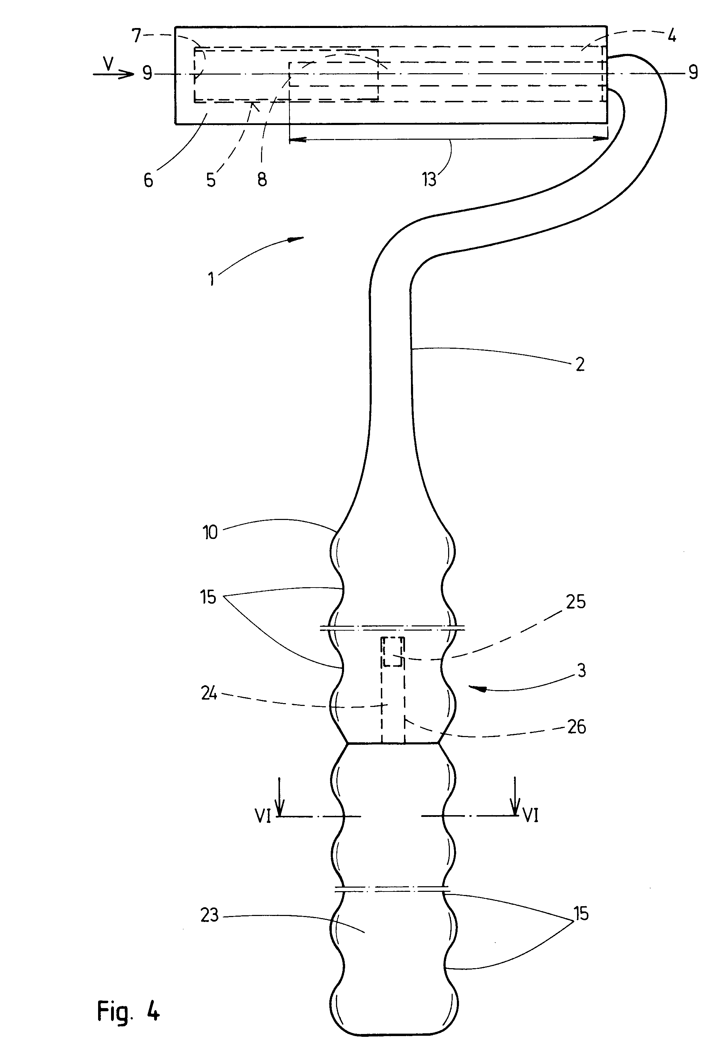

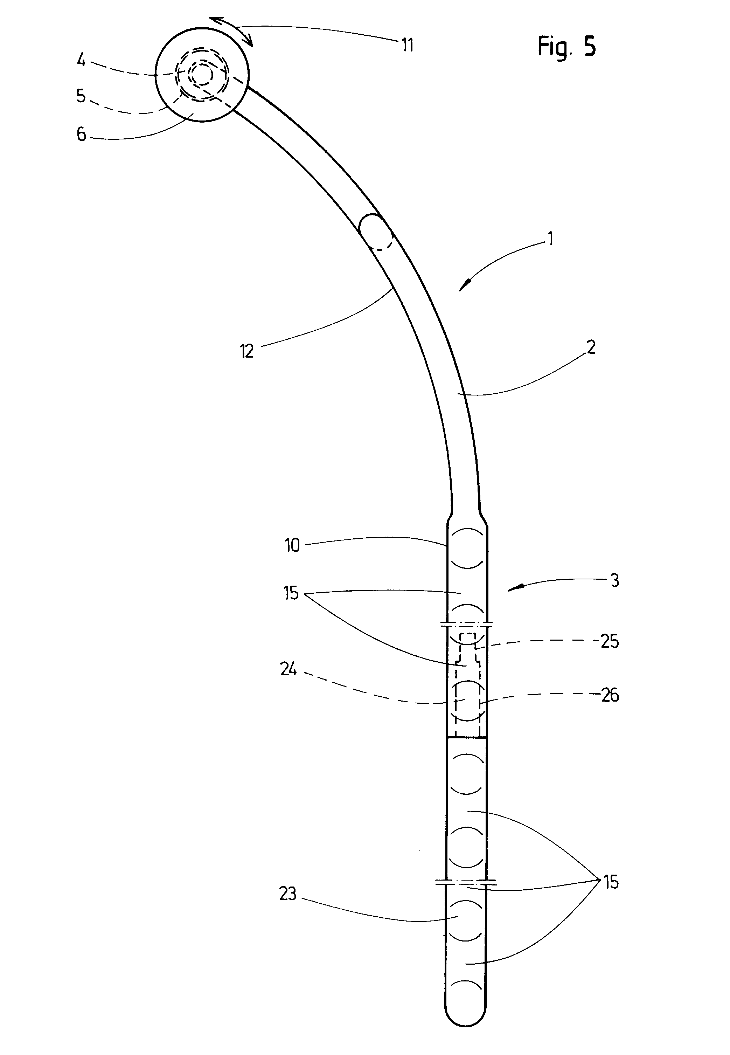

Als Schutz gegen Eintrocknen und Schmutz kann die Rolle 4 mit ihrer Beschichtung 6 von einer Schutzhülle 16 umhüllt werden, die aus zwei Teilen 17, 18 besteht. Die beiden Teile 17, 18 sind einerseits über ein Filmscharnier 19 miteinander verbunden und andererseits mittels in den Teilen 17, 18 eingeformter druckknopfartiger Rasten 20 verschließbar (siehe Fig. 7).

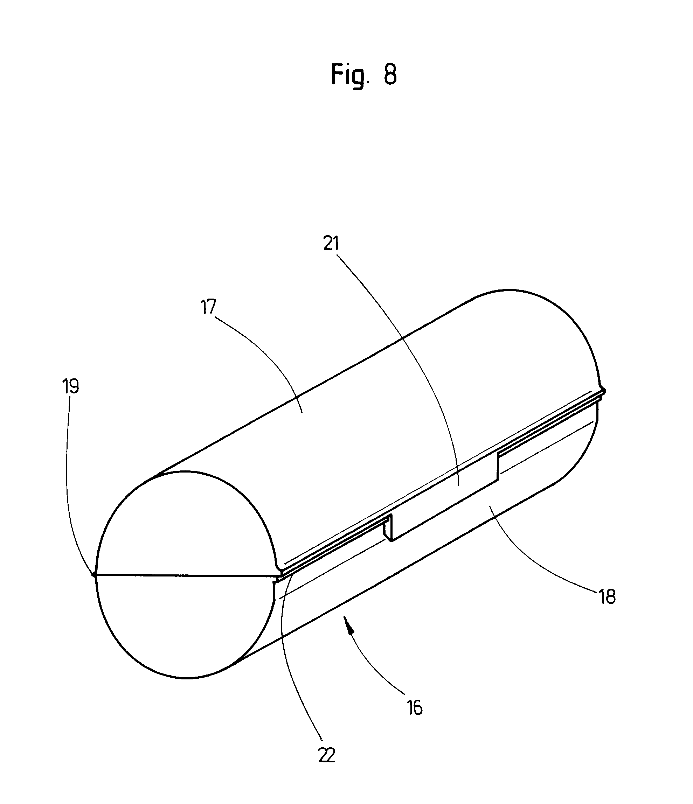

Bei einem anderen Ausführungsbeispiel (Fig. 8) besteht die Schutzhülle 16 ebenfalls aus zwei Teilen 17, 18. Auch hier sind einerseits die beiden Teile 17, 18 über ein Filmscharnier miteinander verbunden. Andererseits jedoch ist an einem 17 der Teile 17, 18 eine Rastnase 21 angeformt, die in eine Hinterschneidung 22 des anderen Teiles 18 einrastet und die Schutzhülle 16 verschließt.

Um auch ein Erreichen von schwer zugänglichen Körperpartien ohne Inanspruchnahme der Hilfe einer weiteren Person zu ermöglichen, ist an das untere Ende des den Handgriff 3 aufweisenden Bügelteils 10 ein Verlängerungsstab 23 ansetzbar. Der Verlängerungsstab 23 weist einseitig einen Steckbolzen 24 mit seitlichen Abflachungen 25 auf, der in eine entsprechende Bohrung 26 am unteren Ende des Handgriffes 3 einsetzbar ist. Die Abflachungen 25 sorgen dafür, daß sich der Verlängerungsstab 23 bzw. der Steckbolzen 24 nicht im Handgriff 3 unkontrolliert dreht.

Der Verlängerungsstab 23 weist ebenfalls die am Bügelteil 10 vorgesehen Griffmulden 15 auf, und es versteht sich als selbstverständlich, daß der Querschnitt des jeweiligen Verlängerungsstabes 23 dem Querschnitt des jeweiligen Handgriffes 3 entspricht.

## Patentansprüche

1. Vorrichtung zum Auftragen und Verteilen von Salben, Lotionen, Flüssigkeiten o.dgl. auf Körperpartien, mit einem Tragbügel, an dessen einem Ende ein Handgriff vorgesehen ist und an dessen anderem freien Ende eine Rolle aufsetzbar und freidrehbar gelagert ist, die an ihrer Umfangsfläche eine Beschichtung trägt, dadurch gekennzeichnet, daß der Tragbügel (2) mit seinem rechtwinklig zur Längsachse (9-9) der Rolle (4) angeordneten und den Handgriff (3) aufweisenden Bügelteil (10) eine der Bewegungsrichtung (11) der Rolle (4) angepaßte Biegung (12) aufweist.

2. Vorrichtung nach Anspruch 1, dadurch gekennzeichnet, daß der Radius der Biegung (12) ca. 150 mm beträgt.

3. Vorrichtung nach Anspruch 1 oder 2, dadurch gekennzeichnet, daß der Tragbügel (2) aus Kunststoff besteht.

4. Vorrichtung nach Anspruch 1 oder 2, dadurch gekennzeichnet, daß der Tragbügel (2) ein Metallbügel (2') ist, der außerhalb des Aufsteckbereiches (13) der Rolle (4) eine Kunststoff-Ummantelung (14) aufweist.

5. Vorrichtung nach einem der Ansprüche 1 bis 4, dadurch gekennzeichnet, daß der Tragbügel (2) bzw. die Kunststoff-Ummantelung (14) an dem rechtwinklig zur Längsachse (9-9) der Rolle (4) angeordneten Bügelteil (10) Griffmulden (15) aufweist.

6. Vorrichtung nach einem der Ansprüche 1 bis 5, dadurch gekennzeichnet, daß der Handgriff (3) einen runden Querschnitt aufweist.

7. Vorrichtung nach einem der Ansprüche 1 bis 5, dadurch gekennzeichnet, daß der Handgriff (3) einen ovalen Querschnitt aufweist.

8. Vorrichtung nach einem der Ansprüche 1 bis 7, dadurch gekennzeichnet, daß die Rolle (4) durch Kraftschluß auf dem Tragbügel (2) gehalten ist, wobei die Rolle (4) zur Drehbewegung eine Drehlagerung (8) aufweist.

9. Vorrichtung nach einem der Ansprüche 1 bis 8, dadurch gekennzeichnet, daß an das untere Ende des den Handgriff (3) aufweisenden Bügelteils (10) ein Verlängerungsstab (23) ansetzbar ist.

10. Vorrichtung nach einem der Ansprüche 1 bis 9, dadurch gekennzeichnet, daß der Verlängerungsstab (23) einseitig einen Steckbolzen (24) mit seitlichen Abflachungen (25) aufweist, der in eine entsprechende Bohrung (26) am unteren Ende des Handgriffes (3) einsetzbar ist.

11. Vorrichtung nach einem der Ansprüche 1 bis 10, dadurch gekennzeichnet, daß der Verlängerungsstab (23) die Griffmulden (15) aufweist.

12. Vorrichtung nach einem der Ansprüche 1 bis 11, dadurch gekennzeichnet, daß die Rolle (4) mit ihrer Beschichtung (6) bei Nichtgebrauch mit einer Schutzhülle (16) versehen ist

13. Vorrichtung nach einem der Ansprüche 1 bis 12, dadurch gekennzeichnet, daß die Schutzhülle (16) aus zwei Teilen (17, 18) besteht, die einerseits über ein Filmscharnier (19) miteinander verbunden sind und andererseits mittels in den Teilen (17, 18) eingeformter druckknopfartiger Rasten (20) verschließbar sind.

14. Vorrichtung nach einem der Ansprüche 1 bis 12, dadurch gekennzeichnet, daß die Schutzhülle (16) aus zwei Teilen (17, 18) besteht, die einerseits über ein Filmscharnier (19) miteinander verbunden sind und andererseits mittels einer in eine Hinterscheidung (22) des einen Teiles (18) einrastenden, an dem anderen Teil (17) angeformten Rastnase (21) verschließbar sind.

15. Vorrichtung nach einem der Ansprüche 1 bis 14, dadurch gekennzeichnet, daß die Beschichtung (6) von der Rolle (4) abnehmbar ist und auf derselben durch Reibschluß gehalten ist.

16. Vorrichtung nach einem der Ansprüche 1 bis 15, dadurch gekennzeichnet, daß an der Umfangsfläche (5) der Rolle (4) einseitig eine Anschlagkante (7) vorgesehen ist.

17. Vorrichtung nach einem der Ansprüche 1 bis 16, dadurch gekennzeichnet, daß die Beschichtung (6) aus Schaumstoff besteht.

18. Vorrichtung nach einem der Ansprüche 1 bis 16, dadurch gekennzeichnet, daß die Beschichtung (6) aus Vlies besteht.

19. Vorrichtung nach einem der Ansprüche 1 bis 16, dadurch gekennzeichnet, daß die Beschichtung (6) aus Flock besteht.
